(19) Europäisches Patentamt
European Patent Office
Office européen des brevets

(11) EP 4 180 989 A1

(12) EUROPEAN PATENT APPLICATION

(43) Date of publication:
**17.05.2023 Bulletin 2023/20**

(21) Application number: **21208123.6**

(22) Date of filing: **15.11.2021**

(51) International Patent Classification (IPC):
***G06F 16/335*** *(2019.01)* ***G06F 16/332*** *(2019.01)*
***G06F 16/33*** *(2019.01)* ***G16H 50/20*** *(2018.01)*

(52) Cooperative Patent Classification (CPC):
**G06F 16/335; G06F 16/3326; G06F 16/3334; G16H 20/00; G16H 50/20; G16H 50/30; G16H 50/70; G16H 70/20**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **VASIST, Santosh Narasimhaswamy**
  **Eindhoven (NL)**
- **BERA, Deep**
  **Eindhoven (NL)**
- **BULUT, Murtaza**
  **Eindhoven (NL)**
- **VAN LIESHOUT, Ron Martinus Laurentius**
  **Eindhoven (NL)**
- **TIEMANN, Christian Andreas**
  **Eindhoven (NL)**
- **MAESSEN, Ralph Theodorus Hubertus**
  **Eindhoven (NL)**
- **SARTOR, Francesco**
  **Eindhoven (NL)**

(74) Representative: **Philips Intellectual Property & Standards**
**High Tech Campus 52**
**5656 AG Eindhoven (NL)**

# (54) METHOD AND APPARATUS FOR PROCESSING DATA

(57) According to an aspect, there is provided a computer implemented method for processing data relating to a subject, the method comprising: generating a first query based on contextual information relating to a subject; receiving, from a user, a second query relating to the subject; and processing the first query and the second query to generate an output comprising at least one of: a request for further input from a user; an indication of clinical information relevant to the subject.

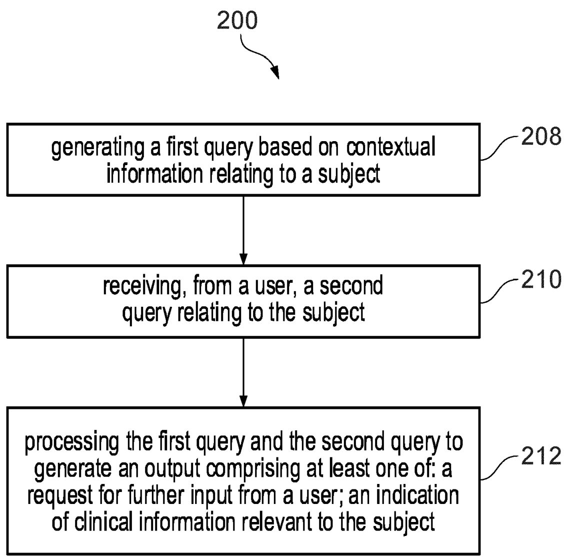


Fig. 2

EP 4 180 989 A1

## Description

FIELD OF THE INVENTION

The invention relates to processing data, and more particularly, processing data relating to a subject.

BACKGROUND OF THE INVENTION

Hospitals often use protocols which are an agreed framework outlining the care that will be provided to patients in a designated area of practice. Typically, each department in the hospital will have hundreds of protocols, with each of these protocols running into many tens of pages. Finding the right protocol from such a huge repository of protocols requires a highly structured query when the search for the appropriate protocol is conducted using a search engine. The search engines used in hospitals to look up protocols can be highly sensitive to keywords input by the users (e.g. caregivers, such as clinicians). This can impact the use of protocols negatively.

The content and the context of protocols often overlap in many situations and even cross through various departments. Often, a caregiver wants to access a particular section of the protocol. However, finding the right information at the right time becomes extremely challenging. This is also one of the hindering factors for the use of protocols. Hence the quality of care, adherence and compliance of care (for example, in the ICU) may all suffer.

SUMMARY OF THE INVENTION

It is desirable to provide improved methods for processing data relating to a subject in order to provide appropriate clinical information relating to a condition of a subject.

According to an aspect, there is provided a computer implemented method for processing data relating to a subject, the method comprising: generating a first query based on contextual information relating to a subject; receiving, from a user, a second query relating to the subject; and processing the first query and the second query to generate an output comprising at least one of: a request for further input from a user; an indication of clinical information relevant to the subject.

This may provide an improved method to help to identify a relevant piece of clinical information, such as a relevant protocol, within a short time period. The method may also reduce the chance of error in patient management and prevent tunnel-vision of the clinician treating the subject. For example, if the relevant protocol is not found at the time of intervention, a user (e.g. a clinician) wouldn’t have any other choice than taking the step which they feel is the best, whereas the above method may present a better alternative solution to the user.

According to an aspect, there is provided a computer program product comprising a computer readable medium, the computer readable medium having computer readable code embodied therein, the computer readable code being configured such that, on execution by a suitable computer or processor, the computer or processor is caused to perform any of the methods described herein.

According to an aspect, there is provided system for processing data relating to a subject, the system comprising: a memory comprising instruction data representing a set of instructions; and a processor configured to communicate with the memory and to execute the set of instructions, wherein the set of instructions, when executed by the processor, cause the processor to: generate a first query based on contextual information relating to a subject; receive, from a user, a second query relating to the subject; and process the first query and the second query to generate an output comprising at least one of: a request for further input from a user; an indication of clinical information relevant to the subject.

These and other aspects will be apparent from and elucidated with reference to the embodiment(s) described hereinafter.

BRIEF DESCRIPTION OF THE DRAWINGS

Example embodiments will now be described, by way of example only, with reference to the following drawings, in which:

Fig. 1 is an illustration of a system according to an example;
Fig. 2 is an illustration of a method according to an example;
Fig. 3 is an illustration of clinical information according to an example;
Fig. 4 is a block diagram illustrating a system according to an example;
Fig. 5 is a block diagram illustrating a part of the system according to an example;
Fig. 6 is a block diagram illustrating a part of the system according to an example;
Fig. 7 is a block diagram illustrating a part of the system according to an example; and

Fig. 8 is an illustration of a clinical decision support system according to an example.

DETAILED DESCRIPTION OF EMBODIMENTS

Turning now to Fig. 1 in some embodiments there is an apparatus 100 for use in processing data relating to a subject, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

The apparatus comprises a memory 104 comprising instruction data representing a set of instructions and a processor 102 (e.g. processing circuitry or logic) configured to communicate with the memory and to execute the set of instructions. Generally, the set of instructions, when executed by the processor, may cause the processor to perform any of the embodiments of the methods as described below.

Embodiments of the apparatus 100 may be for use in processing data relating to a subject. More specifically, the set of instructions, when executed by the processor, cause the processor to: generate a first query based on contextual information relating to a subject; receive, from a user, a second query relating to the subject; and process the first query and the second query to generate an output comprising at least one of: a request for further input from a user; an indication of clinical information relevant to the subject.

The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the data, first query, second query, contextual information, clinical information (for example, a protocol, a portion of a protocol, a medical document, a portion of a medical document, clinician notes, laboratory test result, additional patient data, guidelines, hospital standard operating procedure, medication records, medication leaflets, nurse diaries, clinical trial documents, device operating notes (e.g. instructions, manuals), administrative documents), and/or any other information or data received, calculated or determined by the processor 102 of the apparatus 100 or from any interfaces, memories or devices that are external to the apparatus 100. The processor 102 may be configured to control the memory 104 to store the data, first query, second query, contextual information, clinical information (for example, a protocol, a portion of a protocol, a medical document, a portion of a medical document, clinician notes, laboratory test result, additional patient data, guidelines, hospital standard operating procedure, medication records, medication leaflets, nurse diaries, clinical trial documents, device operating notes, administrative documents).

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may further comprise a display. A display may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method described herein. The apparatus 100 may comprise a battery or other power supply for powering the apparatus 100 or means for connecting the apparatus 100 to a mains power supply.

Turning to Fig. 2, there is a computer implemented method 200 for use in processing data relating to a subject. Embodiments of the method 200 may be performed, for example by an apparatus such as the apparatus 100 described above.

Briefly, in a first step 208, the method 200 comprises generating a first query based on contextual information relating to a subject. In a second step 210, the method comprises receiving, from a user, a second query relating to the subject. In a third step 212, the method comprises processing the first query and the second query to generate an output comprising at least one of: a request for further input from a user; an indication of clinical information relevant to the subject.

Thus, the first query and the second query may be processed to provide an indication of clinical information relevant to the subject. An indication may be, for example, a link to a document (e.g a hyperlink), text from the document, a document reference, a document with a relevant portion highlighted etc.. Alternatively, an output of a request for further input from a user may be made. For example, where it is determined that the second query is very different to the first query, this may indicate that a user has input incorrect information or has been biased in some way when inputting the query, or that the generated query has produced a number of unrelated documents. The first query, which may be based on other information such as vital signs, test results of the subject etc, may therefore indicate that the query input by the user does not consider all aspects of a patient's condition, which may have otherwise led to the wrong clinical information. Therefore, the use of both the first query and the second query may prevent user bias. A query may comprise keywords relating to a condition of a subject.

Where the first query and second query correlate, they may be used to provide an indication of clinical information relevant to the subject to the user. The first query and the second query may be used in conjunction to provide an improved, or augmented, query which is used to generate the indication of clinical information.

Furthermore, a system implementing this method may provide clinical information relevant to the subject quicker than if a user were searching for the relevant information themselves.

The first query may comprise at least one key word relating to a condition of the subject and the second query may comprise at least one key word relating to a condition of a subject, a condition of the subject indicating at least one of: a medical condition of the subject, a medication name, a device name, a procedure name, a severity of a medical condition of the subject. For the second query, for example, if the user needs to access information in a sepsis protocol, and specifically information related to the use of steroids within the sepsis protocol, the second query input by the user may comprise two key words - steroids and sepsis. The first query may comprise medication used by the subject (e.g. warfarin), or vital sign of a subject, such as systolic blood pressure < 100 mmHg, or Heart rate > 120 bpm, and may also include device usage search, such ultrasound Transesophageal Echocardiography (TEE).

The clinical information may be, or comprise, a clinical text, or a portion of a clinical text. The clinical information may comprise at least one of: a protocol; a portion of a protocol; a medical document; a portion of a medical document; clinician notes, laboratory test result, additional patient data, guidelines, hospital standard operating procedure, medication records, medication leaflets, nurse diaries, clinical trial documents, device operating notes, administrative documents. The indication of clinical information may indicate a particular portion of a document as the clinical information.

The processing may comprise determining clinical information relevant to a subject based on a combination of the first query and the second query. A combination of the first query and the second query may be achieved by different means. In one example both queries may have equal weight, but in a different embodiment they may be weighted differently. For instance, the weight can be adjusted based on the accuracy and relevance of clinical information generated as a result of a query, or based on the experience level, and position of clinicians.

In another example, the combination of the first query and the second query may involve dynamically setting the weights for first and second query. In this case, the relevance of the second query to the patient condition can be evaluated, and based on the computed relevance, an appropriate weight can be determined.

The processing may comprise determining clinical information relevant to a subject based on:
the first query, and subsequently selecting from among the determined clinical information based on the second query, or the second query, and subsequently selecting from among the determined clinical information based on the first query. For example, in one case, the first query may be used to narrow down the clinical information, and the second query may be used to select a subset of clinical information from the selection, or in an alternative case, the second query may be used to narrow down the clinical information, and the first query may be used to select a subset of clinical information from the selection. In one example, the narrowing down involves indicating particular protocols, where the clinical information is then indicated from among the information in the protocols.

The processing may further comprise processing the first query and the second query individually to generate a first output of clinical information corresponding to the first query, and a second output of clinical information corresponding to the second query, and wherein the first and second outputs are compared, whereby if the similarity of the first output and second output is less than a threshold, a request for further input from a user is output. Thus, the clinical information corresponding to the first query and the clinical information corresponding to the second query may be compared to determine whether the first query and the second query correspond. As is described above, a user may have a bias which leads them to inputting a query which may be less relevant to the condition of the patient, where the comparison between the two queries may be used to highlight this to the user by requesting further input. The request for further input may also indicate why the request has been made, such as conflict between the output clinical information of the first and second query.

The contextual information may comprise information that is relates to a subject, for example relating to a medical condition of a subject. The contextual information may comprise clinical data relating to a subject. The contextual information may comprise at least one of: an electronic medical record, laboratory test result, medication data, data from a subject monitor, waveforms from a subject monitor, age, gender, clinical observations, data from a ventilator, an ontology database, sensor data, clinician notes, and workflow information, location and/or availability of staff and equipment relating to the patient.

The contextual information may be input to a clinical decision support system (CDSS) to generate the first query. A clinical decision support system may be a system which is capable of predicting or identifying a condition of a subject. The clinical decision support system may be configured to determine a medical condition of the subject. The clinical decision support system may be configured to determine the severity of a medical condition of the subject. For example, the clinical decision support system may take as input contextual information relating to the subject. The contextual information may comprise any of an electronic medical record, laboratory test result, medication data, data from a subject monitor, waveforms from a subject monitor, age, gender, clinical observations, data from a ventilator, an ontology database, sensor data, clinician notes, and workflow information, location and/or availability of staff and equipment relating to the patient. The contextual information may then be processed by a CDSS to generate at least one severity keyword and at least one medical condition keyword (e.g. comprising keywords such as medication etc). Thus, any of the contextual information may be used alone or in combination to determine the medical condition and/or a severity of a medical condition of a subject.

The processing may comprise ranking at least two pieces of clinical information based on at least one of: the relevance to the subject; the urgency of applying the clinical information in relation to a condition of the subject; experience of the user; previous knowledge of the user. Thus, the ranking may be based on how well the clinical information corresponds to the query, and/or may depend on how urgent it is that the user acts on the clinical information. For example, one piece of clinical information may be more relevant overall to the query, but another piece of clinical information may be required to be used immediately in order that a subject's (e.g. a patient's) condition does not worsen. The highest ranking clinical information may be selected as the information to indicate. The indication of at least one piece of clinical information may comprise at least one of: a ranking of at least two pieces of clinical information; the most optimal clinical information. For example, the clinical information may be ranked and then only the most relevant clinical information may be presented to the user. For example, more than one piece of clinical information may be indicated to a user (e.g. with a ranking), or the most optimal clinical information may be presented to a user.

Some (elements of) therapies or procedures to follow may appear in multiple protocols. Finding and selecting the protocol which is most appropriate for the subject being treated may become challenging in these cases. The methods described herein may improve the finding and selection of protocols for the treatment of a subject. The examples below are based on information from guidelines obtained from Wolters Kluwer UpToDate.

An example of a situation in which the methods herein may be used is in the treatment of hypovolemia, a state of abnormally low extracellular fluid in the body. Potential etiologies of hypovolemia include gastrointestinal, renal, skin, haemorrhage, and third-space losses. Depending on the etiology and subject context different strategies exist for treating the volume deficit (which may be captured in different protocols). Supporting the selection of the most appropriate protocol for the given patient may help the care team in defining the optimal treatment. For example, a choice of replacement fluid needs to be made (e.g., crystalloid solution, colloid-containing solution, blood product). Some patients may benefit more from one type over the other. Hyperoncotic starch solutions should, for example, be avoided in critically ill patients. Also, the rate of fluid repletion depends on the patient profile. Another example is the administration of vasopressors in patients who do not sufficiently respond to fluid therapy. In general, these should not be administered since they do not correct the primary problem. However, in patients with refractory shock, vasopressors may be administered. Examples of patient populations with hypovolemia which may need special attention are acute pancreatitis, sepsis, lactic acidosis, acute respiratory distress syndrome, trauma, diabetic ketoacidosis, chronic liver disease. Certain treatment details may be covered in dedicated protocols, such as for sepsis. Selecting the most relevant (part of a) protocol could help the care team to define an optimal treatment.

Another example in which the methods herein may be used is in the management of atrial fibrillation (AF), which is the most common cardiac arrhythmia. In patients with newly diagnosed AF, decisions need to be made regarding the need for anticoagulation and the choice between rate or rhythm control strategies. The strategy highly depends on the patient profile and context. There are patient groups that require specific attention regarding anticoagulant therapy to prevent thromboembolism, e.g., elderly, patients with chronic kidney disease, acute stroke, hyperthyroidism, and cardiac surgery patients, etc. For some of these patient groups a paragraph is included in a guideline document describing the specific treatment recommendations. For some patient groups the document refers to another document.

Fig. 3 provides a representation of a document linking to several other documents, in particular an example of the linking of some of these different documents in Wolters Kluwer UpToDate. A document on atrial fibrillation, anticoagulant therapy to prevent thromboembolism 314 comprises a paragraph on specific patient groups and references to other documents. For different contextual information, such as that the patient is elderly 316, acute stroke 318, hyperthyroidism 320, cardiac surgery 322, long-term antiplatelet therapy 324, different documents are indicated corresponding to each of the aforementioned contextual information, such as "risks and prevention of bleeding with oral anticoagulants" 326, "stroke in patients with atrial fibrillation" 328, "cardiovascular effects of hyperthyroidism, section on "atrial fibrillation"" 330, "atrial fibrillation and flutter after cardiac surgery" 332, and "coronary artery disease patients requiring combined anticoagulant and antiplatelet therapy, section on "efficacy and safety"" 334 respectively.

Different strategies exist for rhythm control in patients with or without structural heart disease. The strategy for the maintenance of sinus rhythm also depends on patient conditions like presence of heart failure, coronary artery disease, hypertension. There is also a dedicated protocol for the management of AF in patients with hypertrophic cardiomyopathy. Overall, a search on 'Atrial fibrillation' in Wolters Kluwer UpToDate results in the identification of more than thirty documents. Hence, supporting the selection of the most appropriate (part of a) protocol for the given subject (e.g. patient) using the methods described herein may help a user to determine the optimal treatment strategy.

The methods described herein may improve the relevance of the protocols fetched for an input query by the user (e.g. caregiver). This may be achieved by combining the clinical information (clinical data) from various sources like EMR (electronic medical records), EHR (electronic health records), PM (patient monitor), etc., together with the input query by the user. An overview of an example system utilizing the methods described herein is illustrated in Fig. 4.

The example illustrated in Fig. 4 comprises a context a Context aware searchkey generation (CSG) block 436, which is configured to auto populate the keywords of a query, based on the contextual information relating to the subject, a query-processing (QP) block 438, which is configured to handle the queries from the user (e.g. a caregiver) and search for clinical information, such as a protocol document, from a database, and a result-sorting (RS) block 440, which is configured to sort the search results in descending order of extent of match of the query with the clinical information (e.g. protocols). In particular, in this example, the context aware searchkey generation block 436 takes data from EMR 442 and data from PM 444 (although it will be appreciated that any clinical data may be used). The CDSS 446 then processes the data to generate an output which is fed into the context awareness query database 448, where the query is generated. The output of the context awareness query database 448 is then output to the query processing block 438. The Query processing block comprises the user query 450 which has been received as input from a user 451 and the output of the context awareness query database 448 which are in this example combined to generate a combined query 452, where the combined query is input to a query engine 454 which processes the combined query to generate an indication of clinical information relevant to the subject. The output of the query engine 454 is input to a results sorting block 440. A ranking engine 456 takes the input from the query engine 454 and takes clinical information (e.g. hospital protocols 458) as input, and outputs shortlisted clinical information (e.g. protocols) which the highest ranking 460, which is then output to the user 451.

An example of the query-processing block 538 is illustrated in Fig. 5. In particular, as is shown in Fig. 5, in this example, the generated query (a first query) generated by the context aware searchkey generation block 536 is input to a corpus retrieval block 562. A protocol pdf database 564 and a protocol XML database 566 are in communication with one another, where the protocol XML database 566 is in communication with a search engine 568. The search engine sends documents to and receives key words from the corpus retrieval 562. The corpus retrieval 562 is configured to send document fragments 570 to a query engine 454. The query engine 554 produces indexed search results 572. The query engine 454 is configured to receive as input the document fragments 570 and the user query 550 (e.g. the second query). The query engine 554 is configured to output unsorted search results 574.

The query-processing block of this example handles the retrieval of protocols (or a section of a protocol) from a database (such as a hospital database comprising protocols). This could be achieved any appropriate way. As an example, the method used herein is a simple method termed a pre-fill method. In the pre-fill method, the search is performed two times, the first time with the first (automatically generated) query in the search engine 568 and the second time with the second (user provided) query in the query engine 554 in a sequential manner. The first search is performed immediately after the generation of the first query from the context aware searchkey generation block 536. The search results are temporarily indexed. (The search may be performed on the protocols stored in XML format, and the corresponding PDF equivalent may be displayed to the user.) The user's query 550 may be used to perform the search in the query engine 554 on the previously stored search results (indexed search results 572). Thus, the processing may comprise determining at least one clinical text information relevant to a subject based on the first query, and subsequently selecting from among the at least one determined clinical text information based on the second query (or based on the second query, and subsequently selecting from among the determined clinical information based on the first query). This method reduces the time taken to fetch the results and can prove to be highly useful in critical care settings.

An example of an alternative query-processing block is illustrated in Fig. 6 which shows a representation of combined query method of query processing, which may mitigate bias.

In particular, as is shown in Fig. 6, in this example, the generated query (a first query) generated by the context aware searchkey generation block 636 is input to a corpus retrieval block 662. A query received from a user (e.g. a second query) is also input to the corpus retrieval block 622. A protocol pdf database 664 and a protocol XML database 666 are in communication with one another, where the protocol XML database 666 is also in communication with a search engine 668. The search engine sends documents to and receives key words from the corpus retrieval 662. The corpus retrieval 662 is configured to send document fragments 670 to a query engine 652 (e.g. a combined query engine). The query engine 652 is configured to output unsorted search results 674.

In the combined query method, both the second query of the user and the first query generated by the context aware searchkey generation block 636 are used as independent search queries. For example, the method may comprise processing the first query and the second query individually to generate a first output of clinical information corresponding to the first query, and a second output of clinical information corresponding to the second query, and wherein the first and second outputs are compared, whereby if the similarity of the first output and second output is less than a threshold, a request for further input from a user is output. The search engine 668 produces clinical information (e.g. document fragments) relevant to the subject based on each of the first query and the second query. In this example, the search results of both are compared for similarity in the query engine 652 (e.g. the combined query block). If the results of both the searches are not similar beyond a threshold, it is determined that there exists a bias in one of the user input or the generated query. In such scenarios, more options may be displayed to the user to choose the most relevant protocol, or further input may be requested from the user. This method may ensure misinterpretation of the context or bias by the generation of a query or a user is minimized and the quality of care is unaffected. It will be appreciated that in some examples, the first generated query and the second user query may be combined before being processed to generate clinical information relevant to the subject.

Referring back to Fig. 4, the Context aware searchkey generation (e.g. query generation) block is linked to, or comprises, a CDSS 446 (clinical decision support system) capable of identifying and/or predicting the patient condition. The CDSS considers data from various sources such as EMR, labs, medication data, patient monitor etc and identifies the medical condition of the patient, and the severity of it (EMR 442 and PM 444 are illustrated here as examples). Parameters like age, gender etc., could also be used to augment the query. This data, further structured with the use of an ontology database for appropriateness in the medical context, is used to automatically populate the query (e.g. in the context awareness query database 448) for the relevant protocol to be searched.

Fig. 7 illustrates an example of the Context aware searchkey generation block. In particular, this Figure illustrates a CDSS 746 which is configured to receive any of EMR 742, laboratory test results (labs) 744, clinical observations 776, ventilator data 778, patient monitor data 780, ontology 781. Thus, contextual information is input to a clinical decision support system. It will be appreciated that this Figure illustrates an example of contextual information that is input to the CDSS, however, any appropriate contextual information that may be usable to establish a medical condition or severity of a medical condition may be used. The CDSS 746 output is used to generate a severity keyword 782 and a medical condition keyword 784. The output medical condition keyword and the severity keyword are used to generate a query 786 (the first query). (As an alternative to the CDSS, natural language processing may be used to process the contextual information (e.g. clinical data) to produce the query.)

In one example the CDSS may be Philips's HSI (Hemodynamic stability index). It considers data such as the age of the patient, waveforms from the patient monitor, labs and ventilation data. Based on these parameters, it outputs an HSI score ranging from 0 to 100 indicating the hemodynamic stability of the subject (e.g. patient). The lower the score, higher the instability. It is also capable of indicating the top contributing factors and a type of shock. Suppose in one example the patient goes into septic shock, and where high temperature is the biggest contributing factor. The HSI identifies this condition with a score of 36, where this score is then used to generate the keyword "moderate" for the query (e.g. as the severity of the condition). The identified condition "septic shock" is the subsequent keyword, followed by "high temperature" as the top contributor, as shown in table 1. The severity keyword is "moderate". Thus the query generated (e.g. the first query) will be "moderate septic shock with high temperature". This query is further used by the query processing block.

| CDSS | Prediction score | Severity | Expected condition | Top contributor |
|---|---|---|---|---|
| HSI | 0-30 | Mild risk | Septic shock | Temperature |
| | 31-60 | Moderate risk | | |
| | 61-100 | High risk | | |
| Search key generated | | Moderate septic shock with high temperature | | |

Table 1. A representation of searchkey generation from the CDSS interpretation

Thus, the clinical data is effectively mined to produce keywords or summaries to reflect the condition of the patient, where these keywords are then used by the query processing block to find protocols and indicate information (e.g. highlight part of protocols) that is the most relevant to the condition of the subject.

Another example of a CDSS is the HPI (Hypotension Prediction Index) from Edwards (see Fig. 8 HYPE trial: hemodynamic diagnostic guidance and treatment protocol). As is illustrated in this Figure, the process is started 888 (e.g. by a user). If the HPI is between 50 and 85, diagnosis of the cause is started 890. If the HPI is greater than 85% or MAP (mean arterial pressure) is less than 65, advice is provided to start treatment in less than two minutes 892. Then, if at least two of $Ea_{dyn}$ has dropped, SVR (Systemic Vascular Resistance) has dropped, and SVV (Stroke volume variation) has increased 894, advice is provided that the most likely cause is vasoplegia 596. Otherwise, if two of $Ea_{dyn}$ has increased, SVR has increased, and SVV has dropped 898, advice is provided that the most likely cause is hypovolemia 8100. Otherwise, if three criteria of SVR is the same as or has increased, the SVV is unchanged and the SV (stroke volume) has dropped 8102, advice is provided that the most likely cause is reduced LV contractility 8104. Otherwise, if no condition is satisfied, the process moves to treat hypotension without advice 5104. In this case, the process moves back to analysis of HPI 890, 892. If in any case advice is provided 896, 8100, 8104, treatment is started by the anaesthetist 8106. Then, an evaluation is made of whether there is a dP/dT increase, and if elastance is normalized 8108. The process then moves back to the start 888.

These sorts of treatment protocols can be used to translate the measurement values into a contextual information source relating to a subject used to generate a query. For example, if HPI>85% and SVR (Systemic Vascular Resistance) & SVV (Stroke volume variation) is unchanged but SV (stroke volume) has dropped the key words corresponding to the generated first query will be 'reduced LV contractility'. For example, the "advice" provided in a protocol (such as that shown in Fig. 8) may be used as the key words.

Referring back to Fig. 4, in the results sorting block 440, the search results may be sorted in the order of their relevance and how well they match with the first query and/or the second query. Any appropriate method may be used to achieve this, such as those shown in Wang, Lidan, Jimmy Lin, and Donald Metzler. "A cascade ranking model for efficient ranked retrieval." Proceedings of the 34th international ACM SIGIR conference on Research and development in Information Retrieval. 2011; Croft, W. Bruce. "Combining approaches to information retrieval." Advances in information retrieval. Springer, Boston, MA, 2002. 1-36; and Bartell, Brian T., Garrison W. Cottrell, and Richard K. Belew. "Automatic combination of multiple ranked retrieval systems." SIGIR'94. Springer, London, 1994. A further method which is outlined in Nisheeth, Computer Science and Engineering IIT Kanpur, https://www.google.com/url?sa=t&rct=j&q=&esrc=s&source=web&cd=&cad=rja&uact=8&ved=2ah UKEwjeyd_iodftAhXLeisKHZy-AccQFjACegQIARAC&url=https%3A%2F%2Fwww.cse.iitk.ac.in%2Fusers%2Fnsrivast%2FHCC% 2Franked%2520retrieval.pdf&usg=AOvVaw2_R2pF8GLjZQJ2wSkcd5OX, retrieved 01/11/2021, which outlines the vector space model. This method is based on two key parameters termed term frequency (tf) and inverse document frequency (idf), where term frequency $tf_{t,d}$ is the measure of the number of times a term t appears in a document d, and document frequency is the measure of the number of documents that contain a term. The method eliminates common terms (like high, low etc) and focusses on rare terms. $df_t$ is the document frequency of t: the number of documents that contain t. The inverse document frequency is an inverse measure of the informativeness of a term, hence the log of this measure - inverse document frequency -gives a direct measure of the informativeness.

$$idf_t = \log_{10} (N/df_t)$$

N is the number of documents. Each query is represented as weighted vector (W) of tf-idf. Each document in the database is also represented as a weighted vector of tf-idf.

$$W_{t,d} = \log(1+tf_{t,d}) * \log10(N/df_t)$$

A dot product of the query (q) and the document (d) weighted vectors results in a cosine similarity score. The results are sorted based on the similarity score.

$$\mathrm{Score}(q,d) = \sum_{t \in q \cap d} tf.\,idf_{t,d}$$

The top results (e.g. 5, 10, 20 results), for example, can be displayed to the user to choose from.

The ranking of the search result may be performed using any appropriate method. Any appropriate parameter may be used to rank the search results in addition to or instead of the ranking algorithms previously explained. The frequency of a protocol being used could be one such parameter. Protocols can be highlighted that have frequently been used in the past for similar patients. A Patient Similarity method can be used to extract similar patients (e.g., in terms of age, gender, condition, PM data, EMR data, etc.) from a database and identify corresponding protocols that were used using any appropriate methods. If desired, a further filtering of the identified protocols could be performed based on the outcome success of patients (i.e., did the usage of certain protocols result in better outcomes). The specialization of the department or the hospital could be another such parameter.

The same ranking procedure may be extended to rank the relevance of certain paragraphs present in a protocol, for example, assuming that a single protocol will have several administration principles depending on different patient conditions. Once the protocols have been ranked based on their relevance to the query, a similar method can be used to highlight or hyperlink the most relevant paragraphs (e.g. provide an indication of clinical information relevant to the subject). An example is the guideline related to anticoagulant therapy in atrial fibrillation patients as described above. This document contains various paragraphs with specific treatment recommendations for different patient groups. There are also paragraphs describing the impact and risks of anticoagulation therapy, which are patient dependent. Highlighting the relevant elements of the document may help caregivers to make optimal clinical decisions.

As mentioned above, the 'Result sorting block' may provide the indication of clinical information as a number of top results determined based on cosine similarity scores. However, when the generated query comprises multiple queries relating to multiple patient conditions, this may be an indication that it is not possible to output a single protocol. In order to prevent tunnel-vision the 'Result sorting block' may either add additional results as output to present to the user, or may use the second user query to update the first query to provide a secondary search result to be communicated to the user as a mixture of the first search result and the second search result.

In a further example, the weight of the factors that caused an item of information to be included in the searchable database may be different. For example, where document 1 was included because of the PM data, document 2 was included because of the EMR data, and document 3 was included because of both EMR and PM data, the document included due to more than one piece of clinical data may be given the highest weighting. The reason for inclusion of a particular document or piece of information may be indicated to the user using labels and/or colors which may be attached to the search results. Also, the corresponding parts of the EMR and PM data may also be made available for the user to view. For example, when a user moves a pointer over a search result, they may be able to see the (part of the) EMR data that was the reason this document was selected.

A user who is a caregiver faces numerous challenges in providing quality care. One such challenge is a high level of cognitive load, especially in critical care units. A similar challenge is bombardment of information from various sources like patient monitors. Interpreting the data under the time constraints in a critical care again increases the cognitive load. This may lead to errors and may affect the quality of care. The load on the caregiver and the information crowdedness on the patient monitors may be calculated using any appropriate method. Then, under high load circumstances, only a number of the highest ranking results (e.g. the top 3 or even one best match result) may be displayed to the caregiver to reduce the time required by the caregiver to interpret the search results and select one for use. Thus, the amount of output of clinical information may be regulated based on the load on the caregiver.

The examples described herein may not only help in identifying the relevant protocol within a short time but also reduce the chances of errors in patient management and prevent tunnel-vision. For example, if the relevant protocol is not found at the time of intervention, caregiver would typically simply take the step which they feel is the best, rather than performing a potentially better action.

In another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

**1.** A computer implemented method for processing data relating to a subject, the method comprising:

generating a first query based on contextual information relating to a subject;
receiving, from a user, a second query relating to the subject; and
processing the first query and the second query to generate an output comprising at least one of: a request for further input from a user; an indication of clinical information relevant to the subject.

**2.** The computer implemented method as claimed in claim 1, wherein the first query comprises at least one key word relating to a condition of the subject and the second query comprises at least one key word relating to a condition of a subject, a condition of the subject indicating at least one of: a medical condition of the subject, a severity of a medical condition of the subject, a medication name, a device name, a procedure name.

**3.** The computer implemented method as claimed in any preceding claim, wherein the processing comprises determining clinical information relevant to a subject based on a combination of the first query and the second query.

**4.** The computer implemented method as claimed in claim 1 or 2, wherein the processing comprises determining clinical information relevant to a subject based on:

the first query, and subsequently selecting from among the determined clinical information based on the second query, or
the second query, and subsequently selecting from among the determined clinical information based on the first query.

**5.** The computer implemented method as claimed in any preceding claim, wherein the processing further comprises processing the first query and the second query individually to generate a first output of clinical information corresponding to the first query, and a second output of clinical information corresponding to the second query, and wherein the first and second outputs are compared, whereby if the similarity of the first output and second output is less than a threshold, a request for further input from a user is output.

**6.** The computer implemented method as claimed in any preceding claim, wherein the contextual information comprises at least one of: an electronic medical record, laboratory test result, medication data, data from a subject monitor, waveforms from a subject monitor, age, gender, clinical observations, data from a ventilator, an ontology database, sensor data, clinician notes, and workflow information, location and/or availability of staff and equipment relating to the patient.

**7.** The computer implemented method as claimed in any preceding claim, wherein the contextual information is input to a clinical decision support system to generate the first query.

8. The computer implemented method as claimed in claim 7, wherein the clinical decision support system is configured to determine a medical condition of the subject.

9. The computer implemented method as claimed in 7 or 8, wherein the clinical decision support system is configured to determine the severity of a medical condition of the subject.

10. The computer implemented method as claimed in any preceding claim, wherein the processing comprises ranking at least two pieces of clinical information based on at least one of: the relevance to the subject; the urgency of applying the clinical information in relation to a condition of the subject; experience of the user; previous knowledge of the user.

11. The computer implemented method as claimed in claim 10, wherein the highest ranking clinical information is selected as the information to indicate.

12. The computer implemented method as claimed in any preceding claim, wherein the indication of at least one piece of clinical information comprises at least one of: a ranking of at least two pieces of clinical information; the most optimal clinical information.

13. The computer implemented method as claimed in claim any preceding claim, wherein the clinical information is at least one of: a protocol; a portion of a protocol; a medical document; a portion of a medical document; clinician notes, laboratory test result, additional patient data, guidelines, hospital standard operating procedure, medication records, medication leaflets, nurse diaries, clinical trial documents, device operating notes, administrative documents.

14. A computer program product comprising a computer readable medium, the computer readable medium having computer readable code embodied therein, the computer readable code being configured such that, on execution by a suitable computer or processor, the computer or processor is caused to perform the method as claimed in any one of claims 1 to 13.

15. A system for processing data relating to a subject, the system comprising:

a memory comprising instruction data representing a set of instructions; and
a processor configured to communicate with the memory and to execute the set of instructions, wherein the set of instructions, when executed by the processor, cause the processor to:

generate a first query based on contextual information relating to a subject;
receive, from a user, a second query relating to the subject; and
process the first query and the second query to generate an output comprising at least one of: a request for further input from a user; an indication of clinical information relevant to the subject.

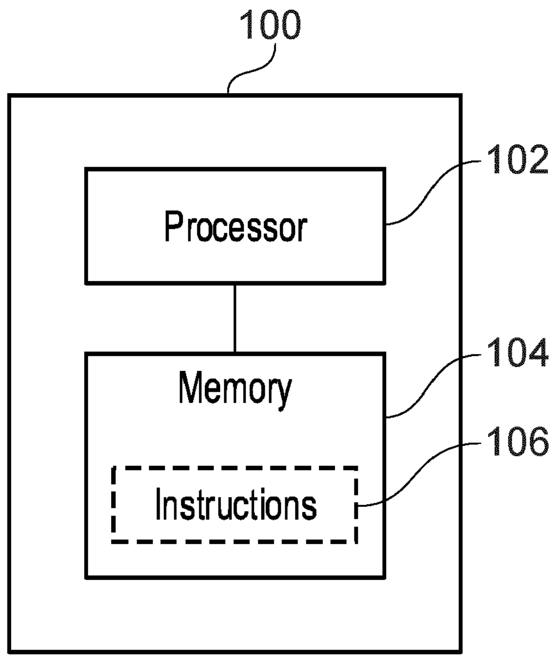


Fig. 1

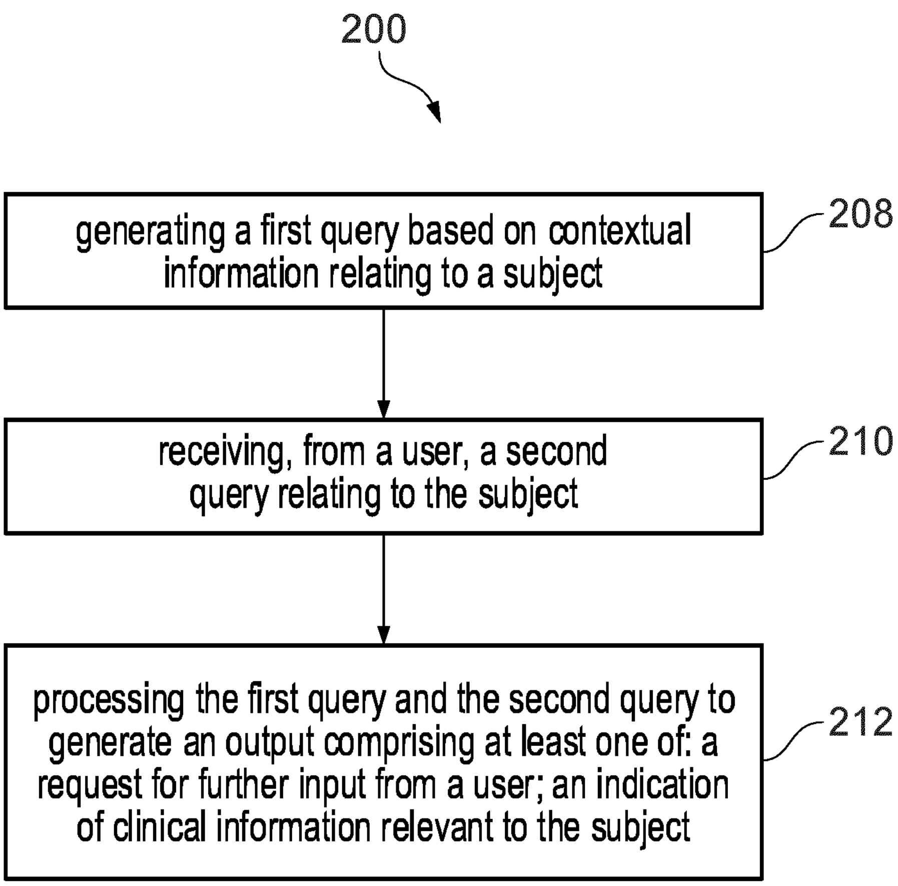
200
generating a first query based on contextual information relating to a subject
208
receiving, from a user, a second query relating to the subject
210
processing the first query and the second query to generate an output comprising at least one of: a request for further input from a user; an indication of clinical information relevant to the subject
212

Fig. 2

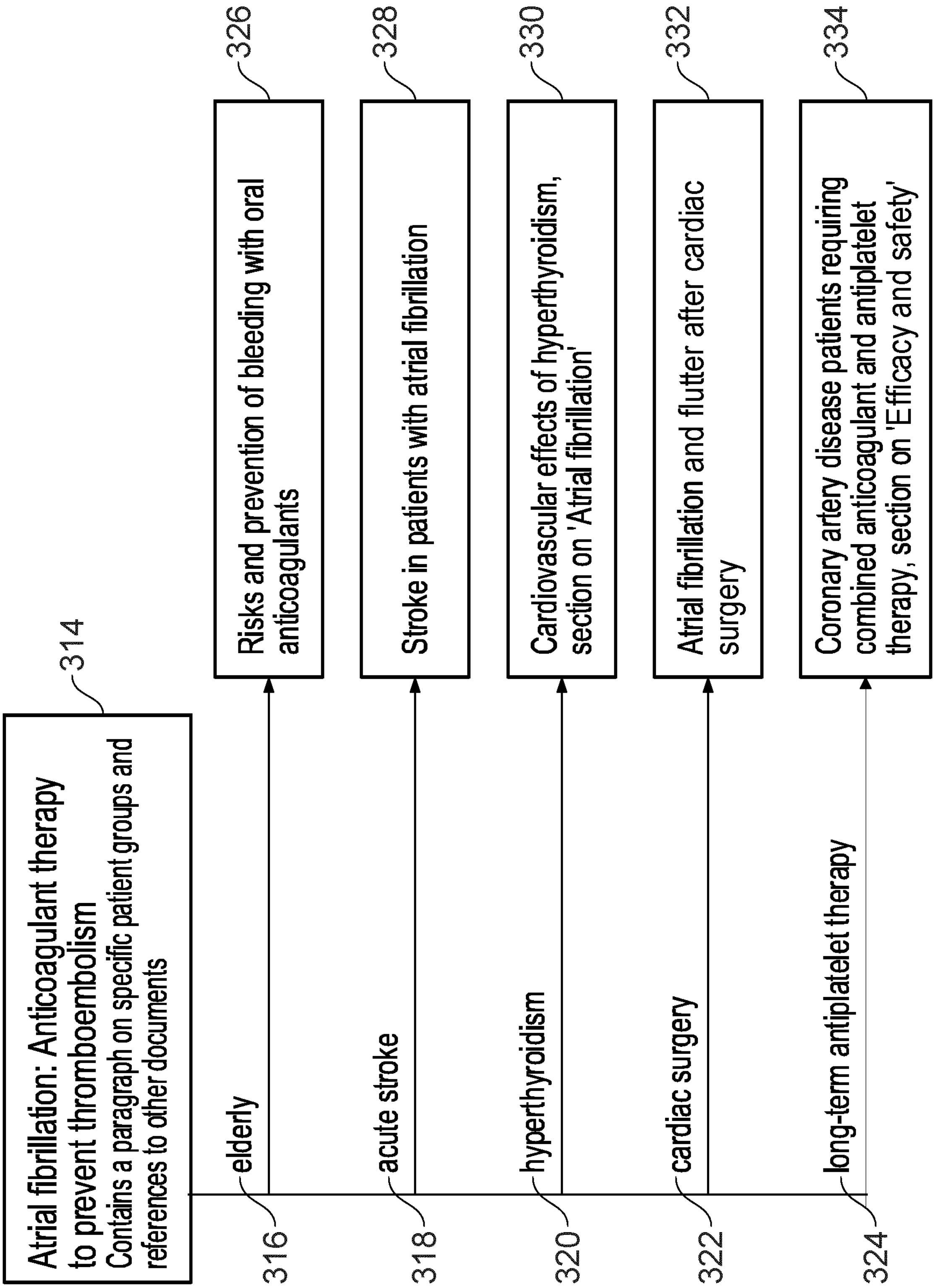
Atrial fibrillation: Anticoagulant therapy to prevent thromboembolism
Contains a paragraph on specific patient groups and references to other documents
314
elderly
acute stroke
hyperthyroidism
cardiac surgery
long-term antiplatelet therapy
316
318
320
322
324
Risks and prevention of bleeding with oral anticoagulants
Stroke in patients with atrial fibrillation
Cardiovascular effects of hyperthyroidism, section on 'Atrial fibrillation'
Atrial fibrillation and flutter after cardiac surgery
Coronary artery disease patients requiring combined anticoagulant and antiplatelet therapy, section on 'Efficacy and safety'
326
328
330
332
334

Fig. 3

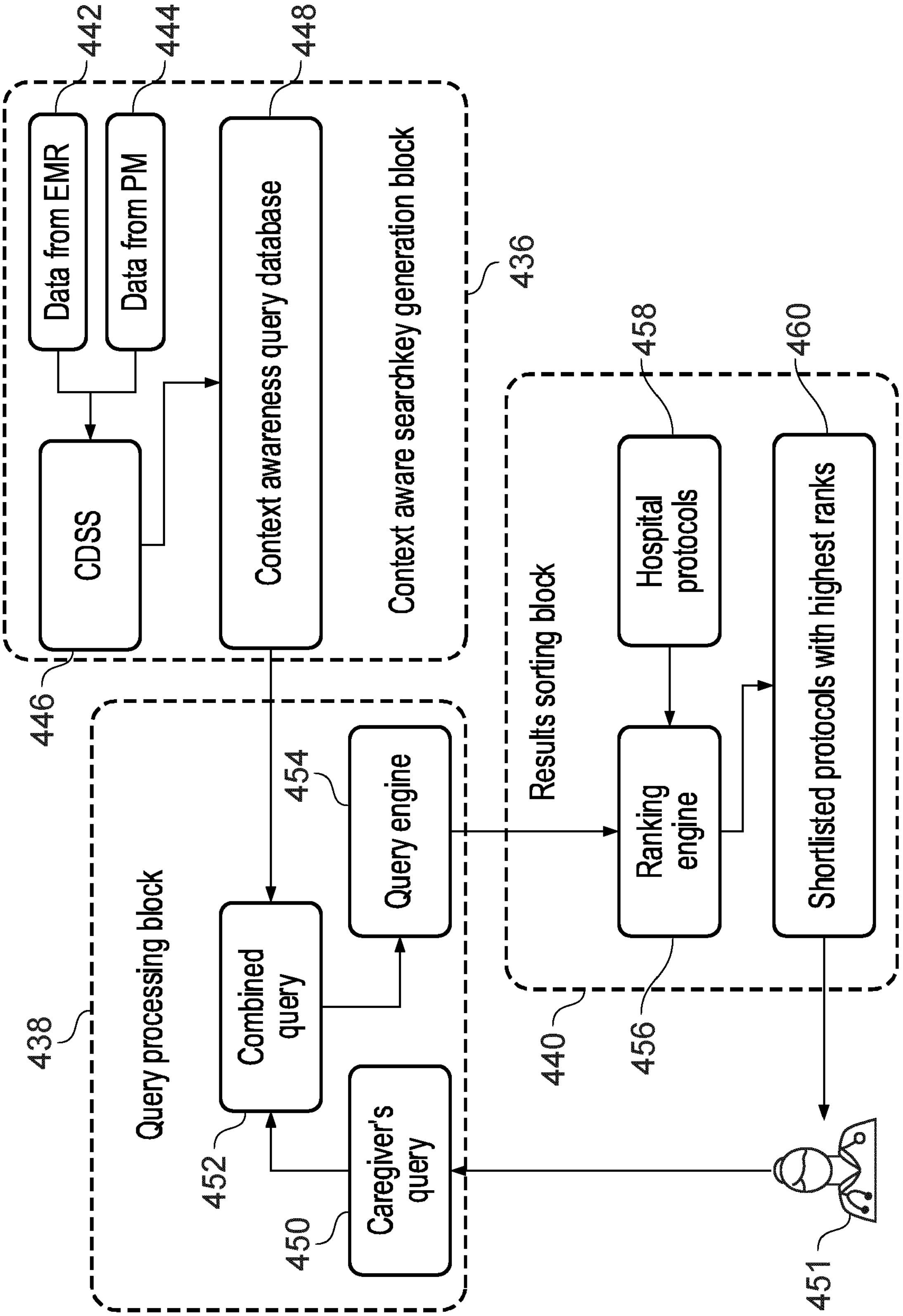
442
444
448
Data from EMR
Data from PM
CDSS
Context awareness query database
Context aware searchkey generation block
436
446
438
Query processing block
Combined query
Query engine
Caregiver's query
450
452
454
Results sorting block
Hospital protocols
Ranking engine
Shortlisted protocols with highest ranks
458
460
440
456
451

Fig. 4

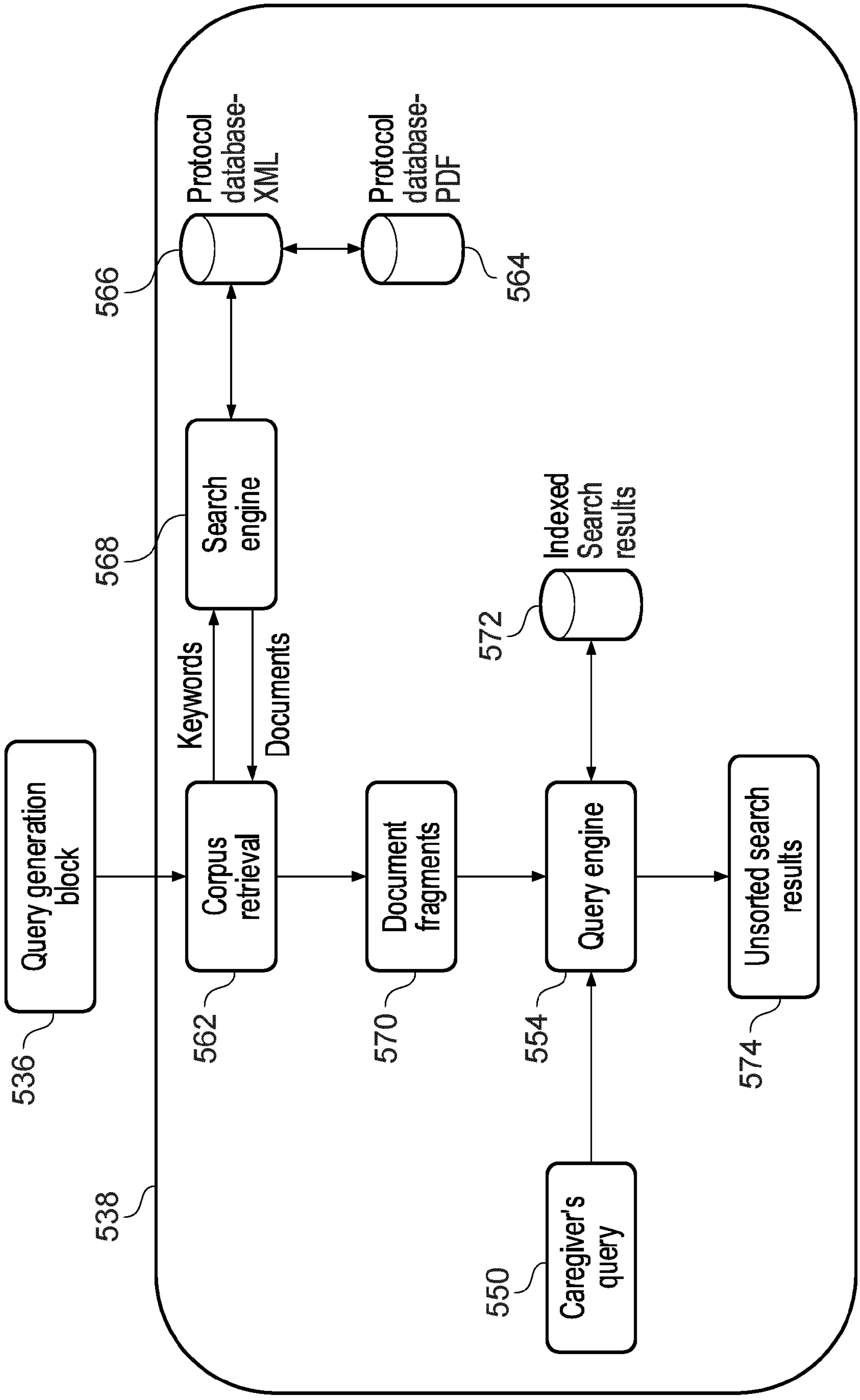
Query generation block
536
538
Corpus retrieval
562
Keywords
Documents
Search engine
568
Protocol database-XML
566
Protocol database-PDF
564
Document fragments
570
Query engine
554
Indexed Search results
572
Caregiver's query
550
Unsorted search results
574

Fig. 5

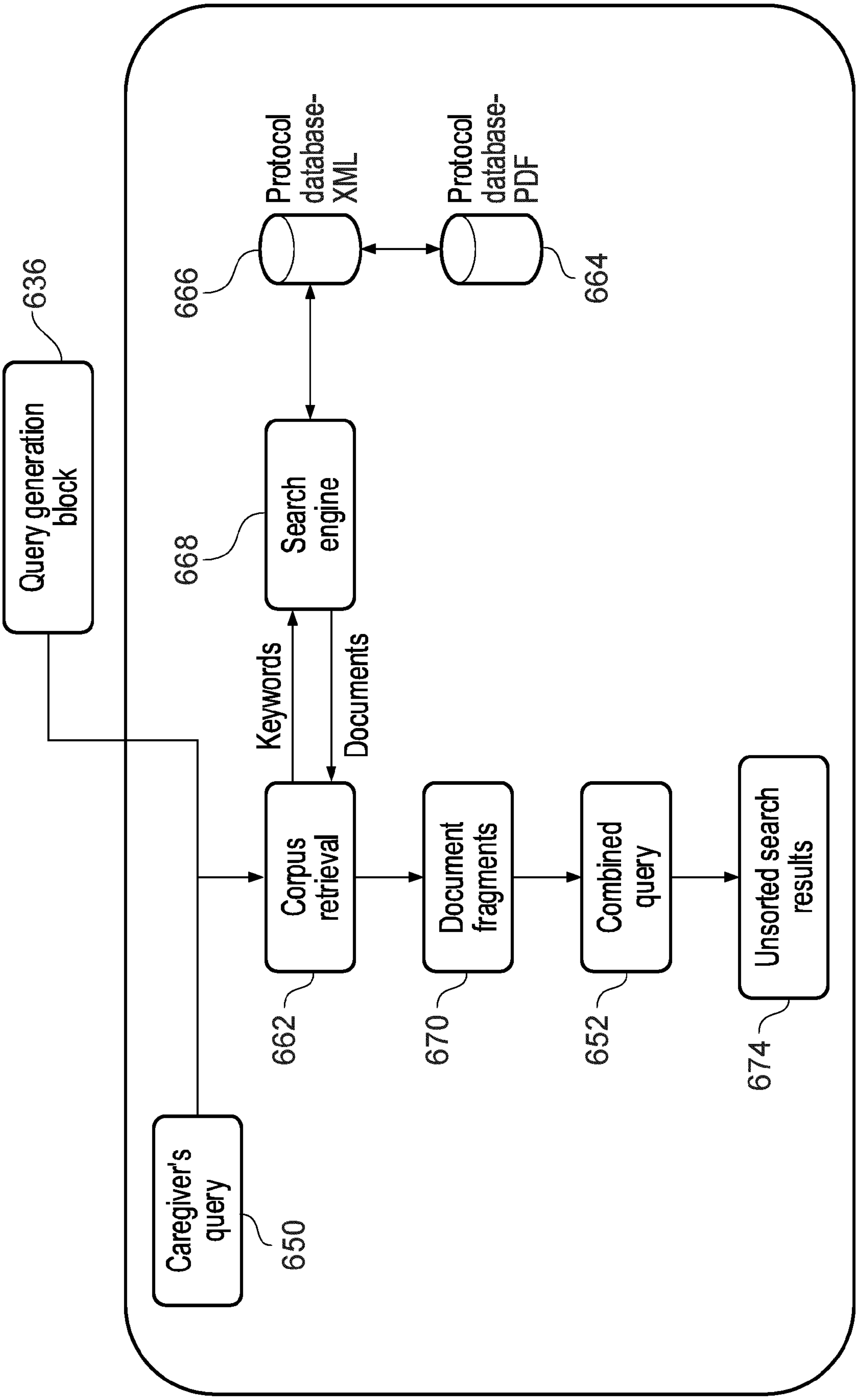
Query generation block
636
Caregiver's query
650
Corpus retrieval
662
Keywords
Documents
Search engine
668
Protocol database-XML
666
Protocol database-PDF
664
Document fragments
670
Combined query
652
Unsorted search results
674

Fig. 6

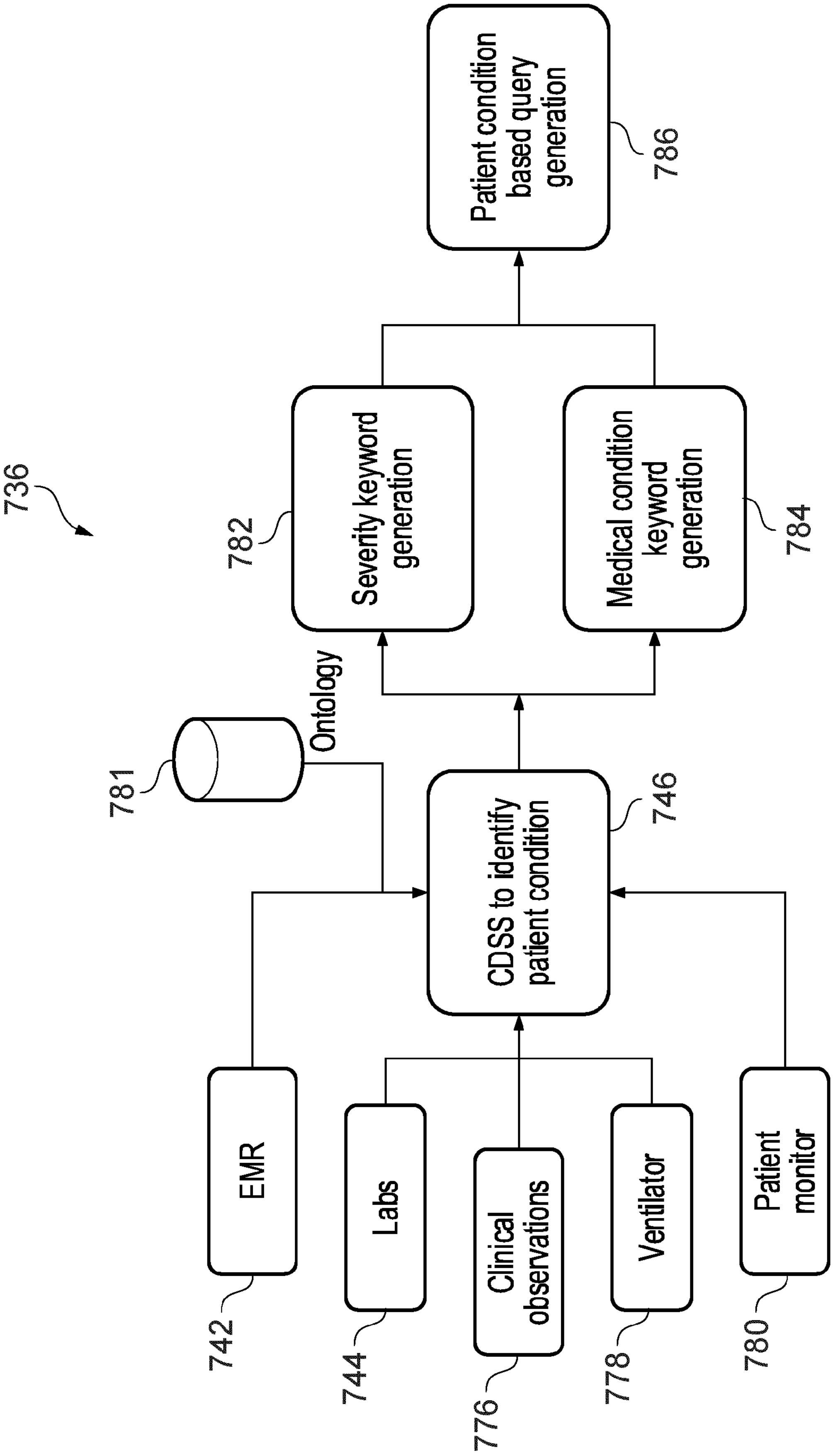
736
EMR
742
Labs
744
Clinical observations
776
Ventilator
778
Patient monitor
780
CDSS to identify patient condition
746
Ontology
781
Severity keyword generation
782
Medical condition keyword generation
784
Patient condition based query generation
786

Fig. 7

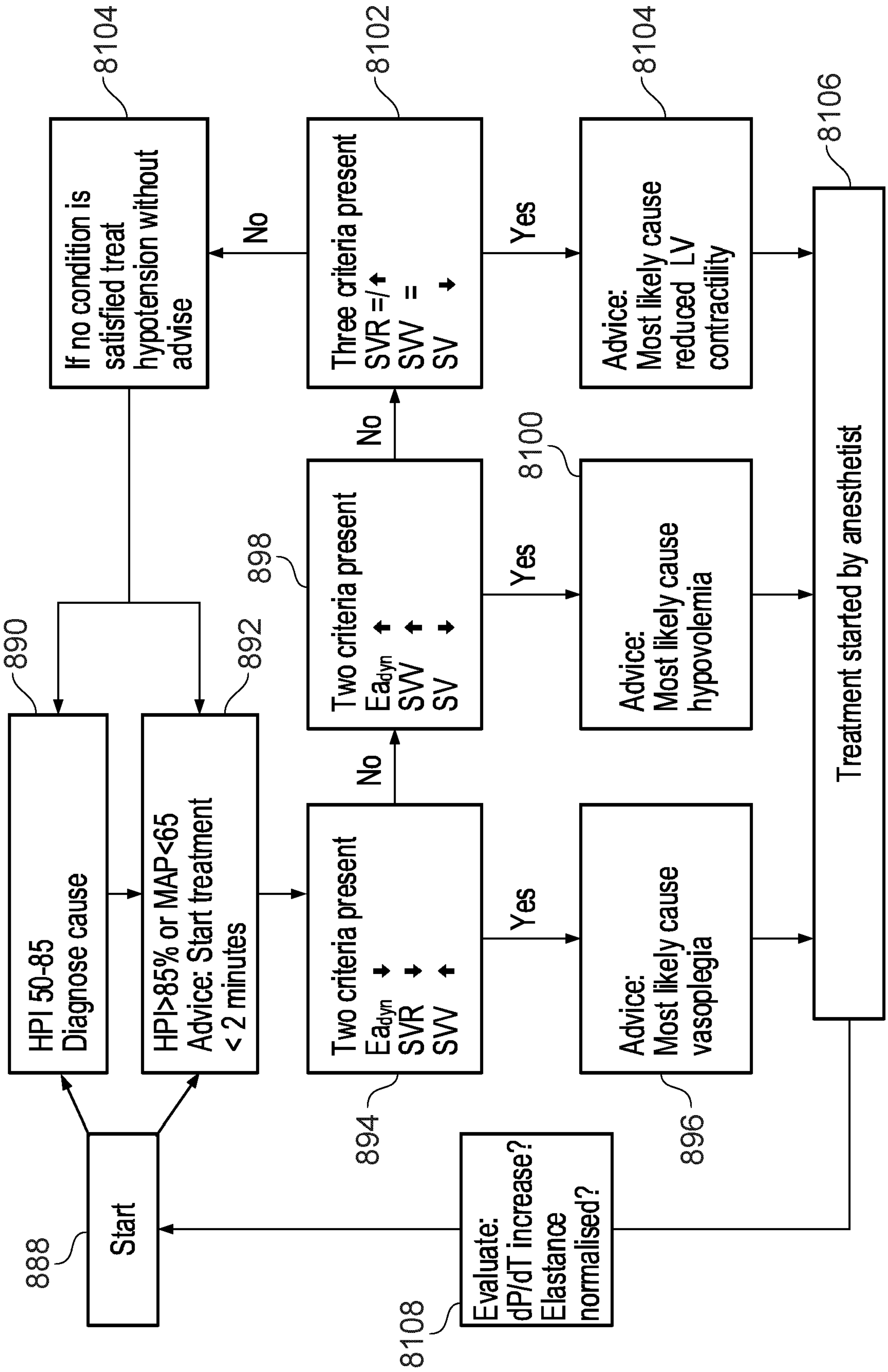

888
Start
890
HPI 50-85
Diagnose cause
892
HPI>85% or MAP<65
Advice: Start treatment
< 2 minutes
894
Two criteria present
$Ea_{dyn}$ ↓
SVR ↓
SW ↑
Yes
No
896
Advice:
Most likely cause
vasoplegia
898
Two criteria present
$Ea_{dyn}$ ↑
SW ↑
SV ↓
Yes
No
8100
Advice:
Most likely cause
hypovolemia
8102
Three criteria present
SVR =/↑
SW =
SV ↓
Yes
No
8104
If no condition is
satisfied treat
hypotension without
advise
8104
Advice:
Most likely cause
reduced LV
contractility
8106
Treatment started by anesthetist
8108
Evaluate:
dP/dT increase?
Elastance
normalised?


Fig. 8

Europäisches Patentamt
European Patent Office
Office européen des brevets

# EUROPEAN SEARCH REPORT

Application Number
EP 21 20 8123

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/196920 A1 (LIANG JENNIFER J [US] ET AL) 12 July 2018 (2018-07-12)<br>* paragraphs [0001], [0092], [0093], [0100] *<br>----- | 1-15 | INV.<br>G06F16/335<br>G06F16/332<br>G06F16/33<br>G16H50/20 |

TECHNICAL FIELDS SEARCHED (IPC)
G06F
G16H

1

The present search report has been drawn up for all claims

| Place of search | Date of completion of the search | Examiner |
|---|---|---|
| The Hague | 17 March 2022 | Rameseder, Jonathan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 21 20 8123

This annex lists the patent family members relating to the patent documents cited in the above-mentioned European search report. The members are as contained in the European Patent Office EDP file on
The European Patent Office is in no way liable for these particulars which are merely given for the purpose of information.

17-03-2022

| Patent document cited in search report | Publication date | Patent family member(s) | Publication date |
|---|---|---|---|
| US 2018196920 A1 | 12-07-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **WANG, LIDAN ; JIMMY LIN ; DONALD METZLER.** A cascade ranking model for efficient ranked retrieval. *Proceedings of the 34th international ACM SIGIR conference on Research*
- *Information Retrieval,* 2011
- Combining approaches to information retrieval. **CROFT, W. BRUCE.** Advances in information retrieval. Springer, 2002, 1-36
- Automatic combination of multiple ranked retrieval systems. **BARTELL, BRIAN T. ; GARRISON W. COTTRELL ; RICHARD K. BELEW.** SIGIR'94. Springer, 1994
- **NISHEETH.** *Computer Science and Engineering IIT Kanpur,* 01 November 2021, https://www.google.com/url?sa=t&rct=j&q=&esrc=s&source=web&cd=&cad=rja&uact=8&ved=2ahUKEwjeyd_iodftAhXLeisKHZy-AccQFjACegQIARAC&url=https%3A%2F%2Fwww.cse.iitk.ac.in%2Fusers%2Fnsrivast%2FHCC%2Franked%2520retrieval.pdf&usg=AOvVaw2_R2pF8GLjZQJ2wSkcd5OX